(19) Europäisches Patentamt
European Patent Office
Office européen des brevets

(11) EP 0 529 976 B1

(12) EUROPEAN PATENT SPECIFICATION

(45) Date of publication and mention of the grant of the patent: **15.05.1996 Bulletin 1996/20**

(51) Int Cl.[6]: **C07D 327/10**, C07D 249/08, A01N 43/26

(21) Application number: **92307669.9**

(22) Date of filing: **21.08.1992**

# (54) 1,3,2-Dioxathiolan-S-oxide derivatives, their use as fungicedes and as intermediates

1,3,2-Dioxathiolan-S-Oxidderivate, ihre Verwendung als Fungizide und als Zwischenprodukte

Dérivés de 1,3,2-dioxathiolan-S-oxyde, leur utilisation comme fongicides et comme intermédiaires

(84) Designated Contracting States:
**BE DE GB NL**

(30) Priority: **23.08.1991 JP 237488/91**

(43) Date of publication of application:
**03.03.1993 Bulletin 1993/09**

(73) Proprietor: **KUREHA CHEMICAL INDUSTRY CO., LTD.**
**Chuo-ku Tokyo 103 (JP)**

(72) Inventors:
- **Kumazawa, Satoru**
  **Iwaki-shi, Fukushima (JP)**
- **Minoguchi, Masanori**
  **Kiyose-shi, Tokyo (JP)**

(74) Representative: **Geering, Keith Edwin**
**REDDIE & GROSE**
**16 Theobalds Road**
**London WC1X 8PL (GB)**

(56) References cited:
**EP-A- 0 357 404** **US-A- 3 395 226**
**US-A- 3 454 597**

- **JOURNAL OF THE CHEMICAL SOCIETY, SECTION C, no. 2, February 1971, Letchworth,**
- **GB, pages 257 - 259 G.P. BLACKBOURN ET AL. 'Studies of the reactions of anhydrosulphites of alpha-hydroxycarboxylic acids. Part VI. Anhydrosulphite synthesis and characterisation'**

EP 0 529 976 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to 1,3,2-dioxathiolan-S-oxide derivatives and to their preparation and use. The 1,3,2-dioxathiolan-S-oxide derivatives according to the present invention are useful as fungicides, and they can be employed as intermediate compounds for the preparation of azolylmethylcyclopentanols known as agricultural and horticultural fungicides and plant growth regulators.

U. S. Patent Nos. 3,395,226 and 3,454,597 disclose that 1,3,2-dioxathiolan-2-oxide and 1,3,2-dioxathiolan-2,2-dioxide derivatives are useful as fungicides.

US-A-4 ,612,322 (EP-A-133,248) discloses fungicidal 1,3,2-dioxathiolan-2-oxide derivatives.

However, the 1,3,2-dioxathiolan-S-oxide derivatives disclosed in the above U.S. Patents have no spiro structure.

We have found that some compounds having 1,3,2-dioxathiolan-S-oxide joined with a cyclopentane ring through a spiro structure are useful as fungicides and that they are useful as intermediate compounds for the preparation of azolylmethylcyclopentanol derivatives of formulae (V) and (VI) below, which are disclosed in US-A-4,863,505 (GB-A-2,180,236) and US-A-4,938,792 (EP-A-267,778).

The present invention provides 1,3,2-dioxathiolan-S-oxide derivatives of formula (I):

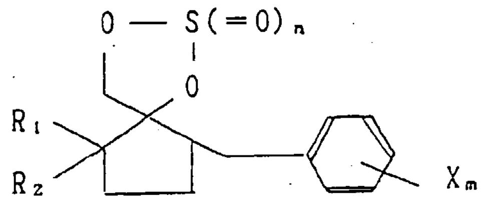

(I)

where $R_1$ and $R_2$ are the same or different and selected from a hydrogen atom and lower alkyl groups;
the or each X, when present, is selected from halogen atoms and cyano, lower alkyl, haloalkyl and phenyl groups;
n is 1 or 2;
m is 0 or an integer from 1 to 5; and when m is greater than 1 any two X's can be the same or different.

The invention also provides a method for the preparation of 1,3,2-dioxathiolan-2-oxide derivative (I-I)

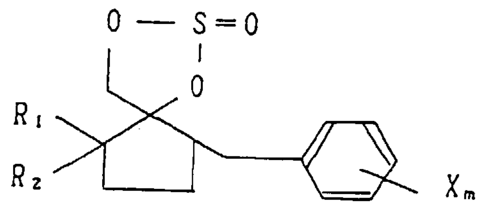

(I-I)

by reacting hydroxymethylcyclopentanol derivative (II)

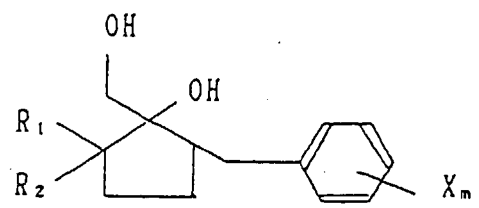

(II)

with thionyl chloride, where $R_1$, $R_2$, X and m are as defined above.

The present invention also provides a method for the preparation of 1,3,2-dioxathiolan-2,2-dioxide derivative (I-II)

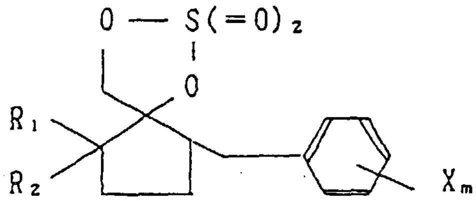

(I-II)

by oxidizing the sulfur atom of 1,3,2-dioxathiolan-2-oxide derivative (I-I).

The present invention further provides a method for the preparation of azolylmethylcyclopentanol derivative (IV) from 1,3,2-dioxathiolan-2-dioxide derivative (I-II)

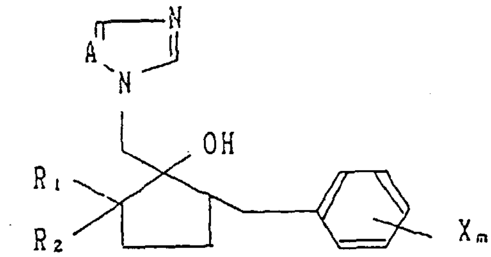

(IV)

by reacting derivative (I-II) with azole derivative (III)

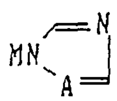

(III)

where $R_1$, $R_2$, X and m are as defined above, A is a nitrogen atom or CH group, and M is an alkali metal or hydrogen atom.

The azolylmethylcyclopentanol derivatives ( IV ) are useful as horticultural fungicides and plant growth regulators.

The term "lower alkyl group" used herein means a monovalent, straight-chained or branched, saturated hydrocarbon residue, preferably of up to 5 carbon atomes (e.g. methyl, ethyl, propyl, iso-propyl, butyl, sec-butyl, tert-butyl and the like). The term "halogen atom" used herein includes for example chlorine, bromine, and fluorine atoms etc. The term "haloalkyl group" used herein includes lower alkyl groups substituted by halogen - for example a fluorine-substituted lower alkyl group such as trifluoromethyl and the like.

The 1,3,2-dioxathiolan-S-oxide derivatives (I) include those of Table 1 below:

Table 1

| Compound Nos. | $R_1$ | $R_2$ | Xm | n |
|---|---|---|---|---|
| I-I-1 | $CH_3$ | $CH_3$ | 4-Cl | 1 |
| I-II-1 | $CH_3$ | $CH_3$ | 4-Cl | 2 |

Note: The term "4-Cl" means substitution of chlorine in the 4-position of the phenyl group.

The 1,3,2-dioxathiolan-S-oxide derivatives (I) may be prepared by the following reaction scheme, which also shows preparation of azolylmethylcyclopentanol derivatives (IV)

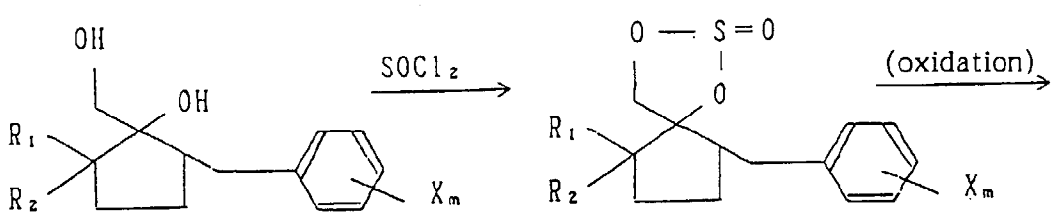


( II )

( I - I )

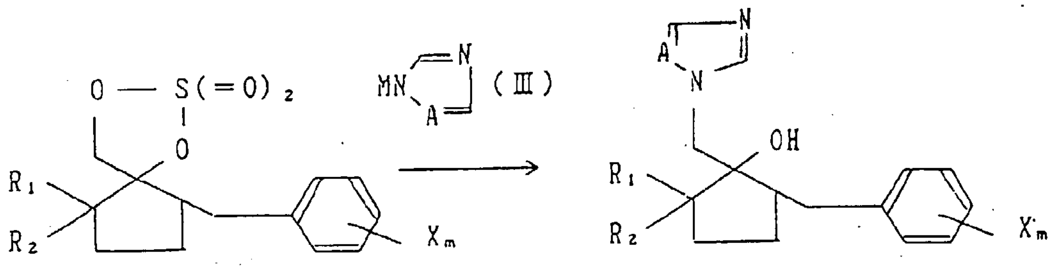

(I − II)

(IV)

The 1,3,2-dioxathiolan-S-oxide derivative(s) (I-I) may be synthesized by reacting hydroxymethylcyclopentanol derivative(s) (II) with thionyl chloride in an organic solvent in the presence of a base.

Suitable organic solvents for the reaction include, for example, haloalkanes such as dichloromethane, chloroform, dichloroethane, etc., aromatic hydro carbons such as toluene, etc., aliphatic hydrocarbons such as hexane, heptane, isooctane, etc., and the like. Suitable bases include, for example, amines such as trimethylamine,triethylamine, N,N-dimethylaniline, N,N-diethylaniline, pyridine, and the like.

The reaction may be carried out at from approximately -40° C to 100° C, preferably from approximately -10° C to 30° C.

After the completion of the reaction, 1,3,2-dioxathiolan-S-oxide derivative (I-I) may be isolated from the reaction mixture by conventional separation methods such as column chromatography.

The 1,3,2-dioxathiolan-S-dioxide derivative(s) (I-II) may be prepared by oxidizing the sulfur atom of 1,3,2-dioxathiolan-S-oxide derivative(s) (I-I) with oxidizing agent in a solvent in the presence of a catalyst.

Suitable oxidising agents include, for example, halogen oxyacid salts such as hypochlorites, periodates etc., and the like.

Suitable catalysts include, for example, ruthenium derivatives such as ruthenium oxide ($RuO_2$) or ruthenium chloride ($RuCl_3$).

Suitable solvents for the oxidation include, for example, polar solvents such as water, lower alcohols (e.g. methanol, ethanol, etc.), acetonitrile, N,N-dimethylformamide, and the like. These solvents may be employed singly or in combination of two or more. When the solvent is employed as a two-phase, water-organic solvent system, the organic solvent may include, for example, haloalkane such as dichloromethane, carbon tetrachloride, etc., or ester such as methyl acetate, ethyl acetate, etc.

The reaction may be carried out at from approximately -40° C to 20° C, preferably from approximately -10° C to 10° C.

The 1,3,2-dioxathiolan-S-dioxide derivative (I-II) may be isolated from the reaction mixture by conventional separation methods.

The 1,3,2-dioxathiolan-S-oxide derivatives (I-I) contain isomers derived from the 2-oxide moiety, and these isomers are part of the invention.

The 1,3,2-dioxathiolan-S-dioxide derivative (I-II) can be reacted with azole derivative (III) above in a solvent to give the azolylmethylcyclopentanol derivatives (V)

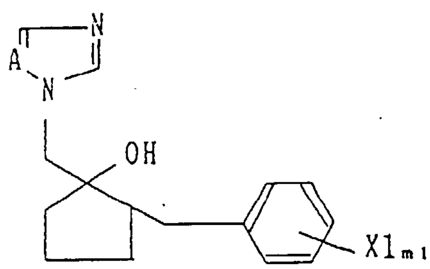

(V.)

(where the or each X1 when present is selected from halogen atoms and lower alkyl, haloalkyl, phenyl, and cyano groups;

A is a nitrogen atom or CH group;

m1 is 0 or an integer from 1 to 5;and

when m1 is greater than 1 any two Xl's can be the same or different); or (VI)

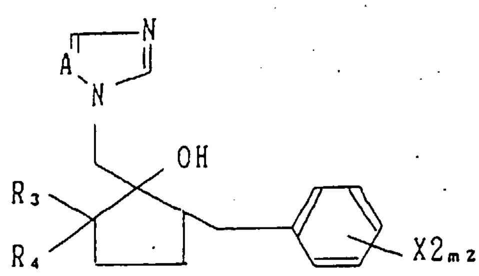

(V.I)

(where $R_3$ and $R_4$ are the same or different and selected from a hydrogen atom and lower alkyl group having from 1 to 5 carbon atoms;
the or each X2, when present, is selected from halogen atoms, alkyl groups having from 1 to 5 carbon atoms and a phenyl group;
A is a nitrogen atom or a CH group; m2 is 0, 1 or 2; and when m2 is 2 the X2's can be the same or different.

Suitable azole derivatives (III) for the reaction include, for example, 1,2,4-triazoles and imidazoles.

Suitable solvents for the reaction include, for example, amides such as N,N-dimethylformamide, N,N-dimethylacetamide, N-methylpyrrolidone, etc., nitriles such as acetonitrile, etc., ketones such as acetone, methyl ethyl ketone, etc., sulfur-containing compounds such as dimethylsulfoxide, sulfolan, etc., ethers such as diethyl ether, tetrahydrofuran, etc., and the like.

The reaction may be carried out at approximately -40° C to 60° C, preferably from approximately -10° C to 30° C.

The azolylmethylcyclopentanol derivatives (IV) can be isolated from the resulting reaction mixture by conventional methods, such as column chromatography.

When 1,3,2-dioxathiolan-S-oxide derivatives (I) are used for fungicidal compositions, they are generally in the form of dust, wettable powder, granules, emulsion and the like together with carriers or other adjuvants. In such a case, the preparations suitably contain one or more compounds (I) in an amount of 0.1 % - 95 % by weight, preferably, 0.5 % - 90 % by weight, and more preferably 2 % - 70 % by weight.

The auxiliary agent to be employed for the preparation may comprise, for example, a carrier, a diluent, a surfactant and the like, which have conventionally been employed as auxiliary agents for the preparation of fungicides.

Suitable carriers in the form of solid material may include, for example, talc, kaolin, bentonite, diatomaceous earth, white carbon, clay, and the like;

diluent in the form of liquid may include, for example, water, xylene, toluene, chlorobenzene, cyclohexane, cyclohexanone,dimethylsulfoxide, dimethylformamide, an alcohol, and the like.

The surfactant is preferably chosen depending upon the form of the preparation; an emulsifiable agent may include, for example, polyoxyethylene alkyl aryl ether, polyoxyethylene sorbitan monolaurate, and the like; a dispersing agent may include, for example, lignin sulfonate, dibutylnaphthalene sulfonate, and the like; and a wetting agent may include, for example, alkyl sulfonate, alkylbenzene sulfonate, and the like.

The above preparations are classified into those which can be used directly, and those which are used after diluting so as to have a suitable concentration with a diluent such as water, etc..

The concentration of compound (I) after dilution is preferably from 0.001 % - 1.0 %.

The application dosage of compound of this invention is suitably 20 g - 5,000 g, preferably 50 g - 1,000 g,per 1 ha of agricultural and horticultural land such as farm, paddy field; fruit garden, hothouse, etc..

It is of course possible to increase and decrease the concentration and the application dosage beyond the above-mentioned ranges, because they depend upon the form of preparations, method of application, place to be used, target crops, etc..

Compounds (I) may be used in combination with other active compounds, such as fungicides, insecticides, miticides, herbicides, and the like.

The present invention is not limited to the following Examples.

The present invention is first illustrated by way of Examples of the preparation of specific compounds.

Preparation Example 1:

Synthesis of 9-[(4-chlorophenyl)methyl]-6,6-dimethyl-1,3,2-dioxathiaspiro[4.4]nonan-2-oxide (Compound No. I-I-1)

Into a 200-ml three-necked flask was charged 100 ml of dichloromethane which in turn was stirred under a nitrogen stream while cooled with ice. In this solution was dissolved 10.75 grams (0.04 mole) of 5-[(4-chlorophenyl)methyl]-1-hydroxy-2,2-dimethylcyclopentanemethanol ($R_1$ = $CH_3$, $R_2$ = $CH_3$ and Xm = 4-Cl in the general formula (II) above),

and 16.22 grams (0.16 mole) of triethylamine was added to the resulting solution.

Into this solution was dropwise added a solution of 7.16 grams (0.06 mole) of thionyl chloride in 20 ml of dichloromethane. The dropwise addition was conducted so that no white fumes were caused to occur and the reaction was carried out between 10° C and 20° C. The solution was then stirred for 15 minutes while cooling it with ice, followed by. adding 100 ml of dichloromethane to the resulting reaction mixture and washing it with 1N-hydrochloric acid and water. The separated organic layer was then dried over anhydrous sodium sulfate and concentrated under reduced pressure, thereby leaving a black oily material in an amount of 15.22 grams.

The oily material was purified by silica gel column chromatography, and the eluate was concentrated to give a crystalline material which in turn was washed with petroleum ether, thereby yielding 9-[(4-chlorophenyl)methyl]-6,6-dimethyl-1,3,2-dioxathia-spiro[4.4]nonan-2-oxide (Compound No. I-I-1) as white crystals in a quantity of 12.37 grams (39.3 mmole).

The percentage yield and the physical properties of the compound (I-I-1) are as follows:

Yield: 98%
mp: 83° C - 85° C (a mixture of two isomers)
MS: $M^+ = 314$ (6%), $M^+ +2 = 316$ (2%)

It was found that the compound prepared hereinabove was a mixture of two isomers so that a portion of the compound was purified by silica gel column chromatography into two isomers. The physical properties of the two isomers were measured and are as follows:

(a) Isomer A:
mp: 103° C to 104° C $^1$H-NMR ($CDCl_3$): $\delta$ 0.97 (s, 6H), 1.17-2.0 (m, 4H), 2.2 - 3.1 (m, 3H), 4.43 (s, 2H), 7.2 (m, 4H)
IR (KBr, $\nu_{max}$): 2968, 2876, 1498, 1202 (S = 0), 1092, 950, 928, 850, 838, 786 $cm^{-1}$

(b) Isomer B:
mp: 118° C to 119° C
$^1$H-NMR ($CDCl_3$): $\delta$ 0.93 (s, 3H), 1.1 (s, 3H), 1.23 - 1.93 (m, 4H), 2.07 - 3.0 (m, 3H), 4.23 (d, 1H, J=8Hz), 4.5 (d, 1H, J=8Hz), 7.03 (d, 2H, J=8Hz), 7.2 (d, 2H, J=8Hz)
IR (KBr, $\nu_{max}$): 2976, 2876, 1496, 1202 (S = 0), 1092, 952, 926, 842, 820, 794 $cm^{-1}$

Preparation Example 2:

Synthesis of 9-[(4-chlorophenyl)methyl]-6,6-dimethyl-1,3,2-dioxathia-spiro[4.4]nonane-2,2-dioxide (Compound No. I-II-1)

Into a 300-ml Erlenmeyer flask was charged 4.38 grams (14 mmole) of the compound (I-I-1) prepared in Example 1 above (as the mixture of the two isomers), and 50 ml of carbon tetrachloride, 50 ml of acetonitrile, and 75 ml of water were added to the compound. After stirring the mixture while cooling with ice, 14.5 mg (0.07 mmole; 0.5 mole%) of $RuCl_3$ and 6.02 grams (28 mmole; 2 mole-eq.) of $NaIO_4$ were added to the mixture, and the resulting mixture was stirred while cooling it with ice.

After 1 hour, the reaction mixture was mixed with 100 ml of ether, thereby separating it into aqueous and organic layers.The aqueous layer was extracted with ether, and the extract was combined with the organic layer, followed by drying it over anhydrous sodium sulfate and concentrating it under reduced pressure, thereby yielding a pale yellow oily material in an amount of 4.47 grams.

The oily material was then purified by silica gel column chromatography, leaving 9-[(4-chlorophenyl)methyl]-6,6-dimethyl-1,3,2-dioxathia-spiro[4.4]nonane-2,2-dioxide (Compound No. I-II-1) as a white crystalline compound in an amount of 4.38 grams (13.5 mmole).

The percentage yield and the physical properties of the compound (I-II-1) are as follows:

Yield: 96.4%
mp: 98° C - 100° C
$^1$H-NMR ($CDCl_3$): $\delta$ 0.97 (s, 3H), 1.2 (s, 3H), 1.33 - 2.0 (m, 4H), 2.1 - 2.6 (m, 1H), 2.6 - 3.27 (m, 2H), 4.43 (d, 1H, J=9Hz), 4.6 (d, 1H, J=9Hz), 7.07 (d, 2H, J=8Hz), 7.27 (d, 2H, J=8Hz)
IR (KBr, $\nu_{max}$): 2980, 2870, 1498, 1478, 1374 (S = 0), 1206 (S = 0), 964, 872, 850 $cm^{-1}$

Preparation Example 3:

Synthesis of 9-[(4-chlorophenyl)methyl]-6,6-dimethyl-1,3,2-dioxathia-spiro[4.4]nonane-2,2-dioxide (Compound No. I-II-1)

Into a 100-ml Erlenmeyer flask was charged 1.14 grams (3.62 mmole) of the compound (I-I-1) prepared in Example 1 above (as the mixture of the two isomers), and 20 ml of acetonitrile and 30 ml of water were added to the compound. After stirring the mixture while cooling with ice, 4.0 mg (0.019 mmole; 0.52 mole%) of $RuCl_3$ and 1.55 grams (7.24 mmole; 2 mole-eq.) of $NaIO_4$ were added to the mixture, followed by stirring it under cooling with ice. The starting material disappeared in 0.5 hour.

To the reaction mixture was added 100 ml of ether, and the reaction mixture was separated into aqueous and organic layers.The aqueous layer was extracted with ether, and the extract was combined with the organic layer, followed by drying it over anhydrous sodium sulfate, filtering out the sodium sulfate, and concentrating it under reduced pressure, thereby yielding a pale yellow solid material.

The solid material was then washed with ether, leaving 9-[(4-chlorophenyl)methyl]-6,6-dimethyl-1,3,2-dioxathiaspiro [4.4]nonane-2,2-dioxide (Compound No. I-II-1) as a white crystalline compound in an amount of 1.13 grams (3.42 mmole).

The percentage yield and the physical properties of the compound (I-II-1) are as follows:

Yield: 94.5%
mp: 98° C - 100° C
$^1$H-NMR ($CDCl_3$): $\delta$ 0.97 (s, 3H), 1.2 (s, 3H), 1.33 - 2.0 (m, 4H), 2.1 - 2.6 (m, 1H), 2.6 - 3.27 (m, 2H), 4.43 (d, 1H, J=9Hz), 4.6 (d, 1H, J=9Hz), 7.07 (d, 2H, J=8Hz), 7.27 (d, 2H, J=8Hz)
IR (KBr, $\nu_{max}$): 2980, 2870, 1498, 1478, 1374 (S = 0), 1206 (S = 0), 964, 872, 850 $cm^{-1}$

Preparation Example 4:

Preparation of cis-5-[(4-chlorophenyl)methyl]-2,2-dimethyl-1-(1H-1,2,4-triazol-1-ylmethyl)-cyclopentanol (IV-1)

Oily sodium hydride (60%; 24.3 mg; 0.6 mmole) was washed with n-hexane, and 3 ml of dimethylformamide (DMF) was added, followed by stirring the mixture at room temperature. To the resulting mixture was added 41.4 mg (0.6 mmole) of 1,2,4-triazole, and the mixture was stirred for 10 minutes, followed by dropwise addition of 1 ml of a DMF solution of 0.1654 gram (0.5 mmole) of the compound (I-II-1) to the resulting mixture.

After stirring the mixture at room temperature for 3 hours, the reaction mixture was poured into a mixture of acetic acid and iced water, and the whole mixture was extracted with ethyl acetate to obtain an organic layer.

After washing the thus obtained organic layer with an aqueous solution of sodium hydrogen carbonate and then with a saline solution, the thus washed organic layer was dried on anhydrous sodium sulfate and then evaporated under reduced pressure to give 0.2026 gram of red oil.

The oily material was then dissolved in 3 ml of tetrahydrofuran (THF), and 0.1 ml of concentrated sulfuric acid and 0.1 ml of water were added to the solution. After the solution was stirred at room temperature for 1 hour, it was neutralized with 1 gram of potassium carbonate and 3 ml of water and then the whole mixture was extracted by ethyl acetate to obtain an organic layer.

After washing the thus obtained organic layer with a saline solution, the thus washed organic layer was dried on anhydrous sodium sulfate and then evaporated under reduced pressure to give 0.1357 gram of yellow oil.

The yellow oil material was then purified by column chromatography with silica gel (Wakogel C-300®; 10 grams) using a hexane:ethyl acetate (5:1) mixture as a developing solution, thereby yielding the compound (IV-1) as a white crystalline substance in an amount of 70.4 mg (0.22 mmole).

The percentage yield and the physical properties of the compound (IV-1) are as follows:

Yield: 44%
mp: 113° C - 114° C
$^1$H-NMR ($CDCl_3$): $\delta$ 0.60 (s, 3H), 1.00 (s, 3H), 1.07 - 1.19 (m, 5H), 2.33 (bs, 2H), 3.53 (bs, 1H), 4.13 (s, 2Hz), 7.06 (d, 2H, J=8Hz), 7.25 (d, 2H, J=8Hz), 8.02 (s, 1H), 8.25 (s, 1H)
IR (KBr, $\nu_{max}$): 3250, 2940, 2850, 1480, 1380, 1262, 1200, 1124, 1080, 1002, 840, 800, 720, 670 $cm^{-1}$

Formulation Examples:

The following examples are directed to formulations or preparations containing 1,3,2-dioxathiolan-S-oxide derivative (I) as an active ingredient.

Formulation Example 1: Dust

| | |
|---|---|
| 9-[(4-chlorophenyl)methyl]-6,6-dimethyl-1,3,2-dioxathia spiro[4.4]nonan-2-oxide (Compound No. I-I-1) | 3 parts by weight |
| Clay | 40 parts by weight |
| Talc | 57 parts by weight |

The above-mentioned ingredients were mixed to prepare a dust.

Formulation Example 2: Wettable Powder

| | |
|---|---|
| 9-[(4-chlorophenyl)methyl]-6,6- dimethyl-1,3,2-dioxathiaspiro[4.4]nonane-2,2-dioxide (Compound No. I-II-1) | 50 parts by weight |
| Lignin sulfonate | 5 parts by weight |
| Alkyl sulfonate | 3 parts by weight |
| Diatomaceous earth | 42 parts by weight |

The above-mentioned ingredients were mixed to prepare a wettable powder. This preparation was used in situ by diluting it with water.

Formulation Example 3: Granules

| | |
|---|---|
| Compound (I-I-1) | 5 parts by weight |
| Bentonite | 43 parts by weight |
| Clay | 45 parts by weight |
| Lignin sulfonate | 7 parts by weight |

The above-mentioned ingredients were mixed and kneaded with addition of water. The mixture was granulated by means of an extrusion granulating machine, followed by drying to obtain granules.

Formulation Example 4: Emulsion

| | |
|---|---|
| Compound (I-II-1) | 20 parts by weight |
| Polyoxyethylene alkyl aryl ether | 10 parts by weight |
| Polyoxyethylene sorbitan monolaurate | 3 parts by weight |
| Xylene | 67 parts by weight |

The above-mentioned ingredients were mixed and dissolved to obtain an emulsion.

Antibacterial Tests:

The 1,3,2-dioxathiolan-S-oxide derivatives (I-I-1) and (I-II-1) were tested for antifungal activities against various plant pathogenic fungi.

Test Procedures:

Each of the 1,3,2-dioxathiolan-S-oxide derivatives (I-I-1) and (I-II-1) was dissolved in dimethylsulfoxide in a suitable concentration, 0.6 ml of the solution was well mixed with 60 ml of a PAS culture medium at about 60°C in a 100 ml conical. flask, and the resultant mixture was poured into Petri dishes and was caused to coagulate, by which plate

culture media containing compound of this invention were obtained.

Plate culture media on which test fungi were previously cultured were punched by a cork borer so as to have a diameter of 4 mm, followed by inoculating on the above-mentioned plate culture medium. After inoculation was carried out, they were incubated for 1 - 3 days at optimum temperature for each fungus and growth of fungi was observed by measuring the diameter of the colony. Hyphae elongation inhibitory rates were determined respectively in accordance with the below described equation:

$$R = 100 (dc - dt)/dc$$

where R = Hyphae elongation inhibitory rate (%)
dc = Diameter of colony on the non-treated plate culture medium
dt = Diameter of colony on the plate culture medium containing the tested compound

The results were ranked in three stages by the following ranking system.

0: R is lower than 50%;
1: R is between 50% and 80%; and
2: R is higher than 80%.

The results against the test fungi are shown in Table 2 below.

Table 2

| | | Test Fungi | | | | |
|---|---|---|---|---|---|---|
| Compound Nos. | Concentration (μg/ml) | H.s. | R.s. | C.l. | S.c. | G.c. |
| I-I-1 | 100 | 2 | 2 | 1 | 2 | 1 |
| I-II-1 | 100 | 2 | 2 | 2 | 2 | 2 |

The abbreviations for the test fungi in Table 2 above are as follows:

H.s.: Helminthosporium sigmoideum
R.s.: Rhizoctonia solani
C.l.: Colletotrichum langenarium
S.c.: Sclerotinia sclerotirum
G.c.: Glomerella cingulata

## Claims

**1.** A 1,3,2-dioxathiolan-S-oxide derivative (I)

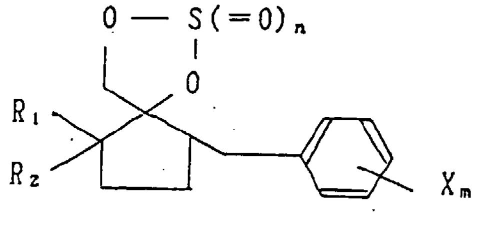

(I)

where $R_1$ and $R_2$ are the same or different and selected from a hydrogen atom and lower alkyl groups;
the or each X, when present, is selected from halogen atoms and cyano, lower alkyl, haloalkyl and phenyl groups; n is 1 or 2;
m is 0 or an integer from 1 to 5; and when m is greater than 1 any two X's can be the same or different.

**2.** A 1,3,2-dioxathiolan-2-oxide derivative according to claim 1 wherein $R_1$ and $R_2$ are methyl, and Xm is a chlorine atom.

3. A 1,3,2-dioxathiolan-2-oxide derivative according to claim 2 wherein the chlorine atom Xm is in the para-position of the phenyl group.

4. A method for the preparation of 1,3,2-dioxathiolan-2-oxide derivative (I-I)

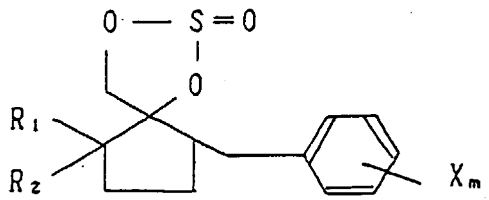

(I-I)

comprising reacting hydroxymethylcyclopentanol derivative (II)

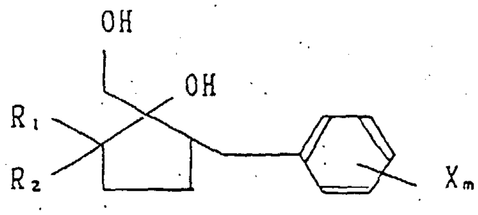

(II)

with thionyl chloride,

where $R_1$ and $R_2$ are the same or different and selected from a hydrogen atom and lower alkyl groups;
the or each X, when present, is selected from halogen atoms and cyano, lower alkyl, haloalkyl and phenyl groups; n is 1 or 2;
m is 0 or an integer from I to 5; and when m is greater than 1 any two X's can be the same or different.

5. A method for the preparation of 1,3,2-dioxathiolan-2,2-dioxide derivative (I-II)

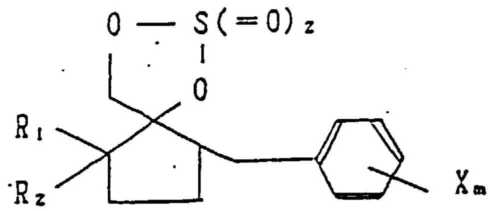

(I-II)

comprising oxidizing the sulfur atom of 1,3,2-dioxathiolan-2-oxide derivative (I-I) as defined in claim 4.

6. A method for the preparation of azolylmethyl-cyclopentanol derivative (IV)

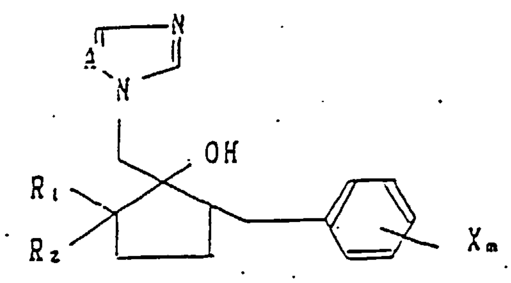

(IV)

26

(where $R_1$, $R_2$, X and m are as defined in claim 4 and A is a nitrogen atom or CH group),comprising reacting 1,3,2-dioxathiolan-2,2-dioxide derivative (I-II) as defined in claim 5 with azole derivative (III)

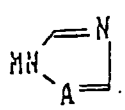

(III)

(where M is an alkali metal or hydrogen atom and A is as defined above).

7. A fungicide containing, as an active ingredient, 1,3,2-dioxathiolan-S-oxide derivative according to any of claims 1 to 3.

## Patentansprüche

1. 1,3,2-Dioxathiolan-S-oxid-Derivat (I)

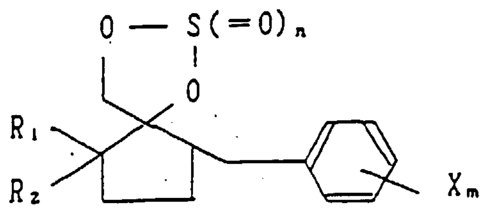

(I)

wobei $R_1$ und $R_2$ gleich oder verschieden sind und ausgewählt sind aus einem Wasserstoffatom und niederen Alkylgruppen;

das oder jedes vorhandene X ausgewählt ist aus Halogenatomen und Cyano-, Niederalkyl-, Halogenalkyl- und Phenylgruppen; n 1 oder 2 ist;
m 0 oder eine ganze Zahl von 1 bis 5 ist und wenn m größer als 1 ist, beliebige zwei X gleich oder verschieden sein können.

2. 1,3,2-Dioxathiolan-2-oxid-Derivat nach Anspruch 1, wobei $R_1$ und $R_2$ Methyl sind und Xm ein Chloratom bedeutet.

3. 1,3,2-Dioxathiolan-2-oxid-Derivat nach Anspruch 2, wobei sich das Chloratom Xm in p-Stellung der Phenylgruppe befindet.

4. Verfahren zur Herstellung eines 1,3,2,-Dioxathiolan-2-oxid-Derivats (I-I)

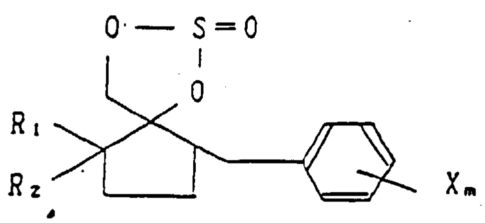

(I-I)

25

umfassend die Umsetzung eines Hydroxymethylcyclopentanol-Derivats (II)

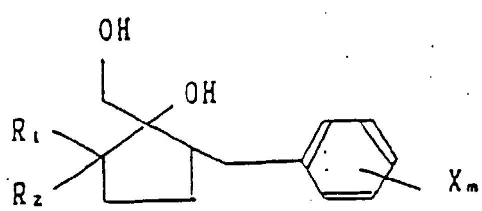

(II)

mit Thionylchlorid, wobei

wobei $R_1$ und $R_2$ gleich oder verschieden sind und ausgewählt sind aus einem Wasserstoffatom und niederen Alkylgruppen;
das oder jedes vorhandene X ausgewählt ist aus Halogenatomen und Cyano-, Niederalkyl-, Halogenalkyl- und Phenylgruppen; n 1 oder 2 ist;
m 0 oder eine ganze Zahl von 1 bis 5 ist und wenn m größer als 1 ist, beliebige zwei X gleich oder verschieden sein können.

5. Verfahren zur Herstellung eines 1,3,2-Dioxathiolan-2,2-dioxid-Derivats (I-II)

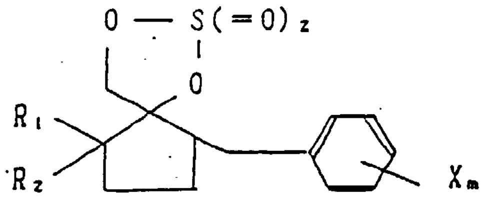

(I-II)

umfassend das Oxidieren des Schwefelatoms des 1,3,2-Dioxathiolan-2-oxid-Derivats (I-I), wie in Anspruch 4 definiert.

6. Verfahren zur Herstellung eines Azolylmethylcyclopentanol-Derivats (IV)

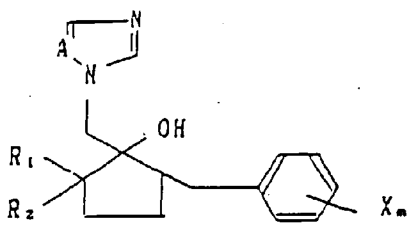

(IV)

(wobei $R_1$, $R_2$, X und m wie in Anspruch 4 definiert sind und A ein Stickstoffatom oder eine CH-Gruppe ist) umfassend die Umsetzung eines 1,3,2-Dioxathiolan-2,2-dioxid-Derivats (I-II), wie in Anspruch 5 definiert, mit einem Azolderivat (III)

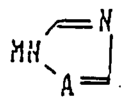

(III)

(wobei M ein Alkalimetall oder Wasserstoffatom ist und A wie oben definiert ist).

7. Fungizid, enthaltend als Wirkstoff ein 1,3,2-Dioxathiolan-S-oxid-Derivat nach einem der Ansprüche 1 bis 3.

**Revendications**

1. Dérivé de 1,3,2-dioxathiolane-S-oxyde (I)

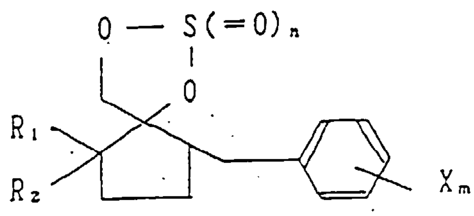

(I)

où

$R_1$ et $R_2$ sont identiques ou différents et sont choisis parmi un atome d'hydrogène et des groupes alkyle inférieur,
le ou chaque X, lorsqu'il est présent, est choisi parmi des atomes d'halogène et des groupes cyano, alkyle inférieur, halogénoakyle et phényle,
n est égal à 1 ou 2,
m est égal à 0 ou à un nombre entier de 1 à 5, et lorsque m est plus grand que 1, deux X quelconques peuvent être les mêmes ou différents.

**2.** Dérivé de 1,3,2-dioxathiolane-2-oxyde suivant la revendication 1, caractérisé en ce que $R_1$ et $R_2$ représentent du méthyle et Xm un atome de chlore.

**3.** Dérivé de 1,3,2-dioxathiolane-2-oxyde suivant la revendication 2, caractérisé en ce que l'atome de chlore Xm est dans la position para du groupe phényle.

**4.** Procédé de préparation de dérivé de 1,3,2-dioxathiolane-2-oxyde (I-I)

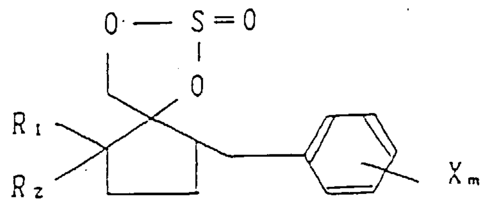

(I-I)

comprenant une réaction, avec du chlorure de thionyle, d'un dérivé d'hydroxyméthylcyclopentanol (II)

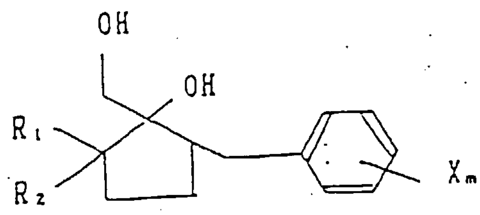

(II)

où

$R_1$ et $R_2$ sont identiques ou différents et sont choisis parmi un atome d'hydrogène et des groupes alkyle inférieur,
le ou chaque X, lorsqu'il est présent, est choisi parmi des atomes d'halogène et des groupes cyano, alkyle inférieur, halogénoalkyle et phényle,
n est égal à 1 ou 2,
m est égal à 0 ou à un nombre entier de 1 à 5, et, lorsque m est plus grand que 1, deux X quelconques peuvent être les mêmes ou différents.

**5.** Procédé de préparation de dérivé de 1,3,2-dioxathiolane-2,2-dioxyde (I-II)

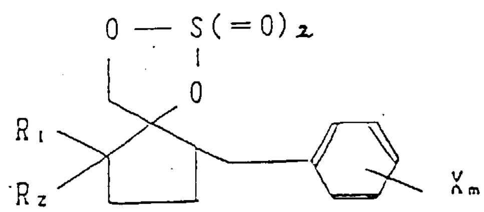

(I-II)

comprenant une oxydation de l'atome de soufre du dérivé de 1,3,2-dioxathiolane-2-oxyde (I-I) tel que défini dans la revendication 4.

**6.** Procédé de préparation de dérivé d'azolylméthylcyclopentanol (IV)

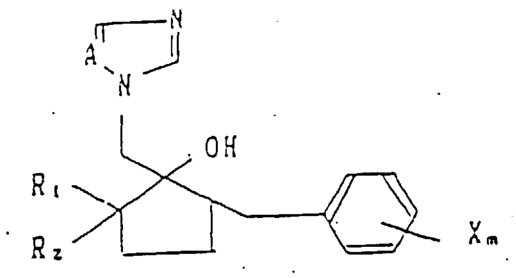

(IV)

où

$R_1$, $R_2$, X et m sont tels que définis dans la revendication 4, et
A est un atome d'azote ou un groupe CH, comprenant une réaction d'un dérivé de 1,3,2-dioxathiolane-2,2-dioxyde (I-II), tel que défini dans la revendication 5, avec un dérivé azole (III)

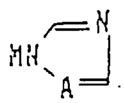

(III)

où
M est un métal alcalin ou un atome d'hydrogène et
A est tel que défini ci-dessus.

**7.** Fongicide contenant, comme composant actif, un dérivé de 1,3,2-dioxathiolane-S-oxyde suivant l'une quelconque des revendications 1 à 3.